(19) **Europäisches Patentamt**

**European Patent Office**

**Office européen des brevets**

(11) **EP 1 471 848 B1**

(12) **EUROPEAN PATENT SPECIFICATION**

(45) Date of publication and mention
of the grant of the patent:
**17.05.2006 Bulletin 2006/20**

(21) Application number: **02806050.7**

(22) Date of filing: **08.01.2002**

(51) Int Cl.:
***A61B 18/26*** (2006.01)

(86) International application number:
**PCT/IL2002/000018**

(87) International publication number:
**WO 2003/057061 (17.07.2003 Gazette 2003/29)**

(54) **ULTRASONIC TRANSDUCER PROBE**

ULTRASCHALLWANDLERSONDE

SONDE DE TRANSDUCTEUR ULTRASONIQUE

(84) Designated Contracting States:
**AT BE CH CY DE DK ES FI FR GB GR IE IT LI LU MC NL PT SE TR**

(43) Date of publication of application:
**03.11.2004 Bulletin 2004/45**

(60) Divisional application:
**06111398.1**

(73) Proprietor: **Bio Scan Ltd.**
**20692 Yokneim Eilit (IL)**

(72) Inventors:
• **AHARONI, Abraham**
 **76346 Rehovot (IL)**
• **STURLESI, Gideon, E.**
 **20112 M.P. Bikat Beit Hakerem (IL)**
• **COTER, Florin**
 **33724 Haifa (IL)**

(74) Representative: **van Westenbrugge, Andries**
 **Nederlandsch Octrooibureau**
 **Postbus 29720**
 **2502 LS Den Haag (NL)**

(56) References cited:
**WO-A-00/33913**   **WO-A-91/10403**
**DE-A- 2 412 690**   **US-A- 4 844 585**
**US-A- 5 944 687**

## Description

The present invention relates to the field of probes including ultrasonic transducers that are powered and/or controlled using non-electrical transmission methods.

Small cross-section catlieters having ultrasound capability at or adjacent to their tips are known in the art. However, transmission of electrical power and/or signals through such thin catheters challenges the design and constrains the ability to reduce the cross-section of the devices. Consequently, several suggestions to transmit power to (and receive signals from) the tip of the catheter using optical waves and convert the optical waves into ultrasonic waves using a suitable transducer, are recorded in the art.

The phenomenon of conversion of electro-magnetic radiation to ultrasound is well established. Of the different conversion modes of clectro-magnetio radiation to ultrasound conversion in the thermo-elastic regime is of primary, but not solitary, interest in this description. In the thermo-elastic regime, a portion of the electro-magnetic radiation absorbed in a target material heats up a region within the target material. Provided the rate of heat deposition is larger than the rate of its dissipation away from the radiated region, the region experiences an increase in its temperature. The resulting thermal stress generates an acoustic disturbance propagating away from the heated region. The rate of heat deposition, as determined from the temporal and spatial parameters of the irradiation wavefront, the rate of dissipation of the heat away from the heated region, and the spatial distribution of temperature in the heated region and the physical properties of the target material determine the characteristics of the resulting acoustic signal.

German laid open patent DE 24 12 690 describes an absorber coupled to a waveguide that absorbs and converts electromagnetic radiation transmitted along the waveguide by an electromagnetic source.

US patent 5,944,687 uses a transducer comprising a fluid reservoir at the tip of the catheter. The fluid is heated by a pulse of laser light transmitted through the catheter. When the heated fluid expands it canses a cap (or bellows) on the fluid reservoir to move. The illumination is transient, and after the light is interrupted, the fluid contracts and the cap retracts.

US patent 6,022,309 describes a different implementation, in which working fliud is conveyed to outside the catheter. Once outside, the fluid is irradiated with pulsed laser light and converts the laser light into ultrasound radiation. Therefore, the ultrasound radiation is generated outside the confines of the catheter.

US patent 5,254,112 describes a catheter in which pulsed laser light hits a target that allegedly generates ultrasound radiation in a direction perpendicular to the target's surface, counter-incident to the light energy. The targets described are metallic. This catheter can allegedly also transmit a high power laser, that is reflected to propagate in the same general direction as the ultrasound radiation, to optically ablate plaque in the vicinity of the catheter. The patent claims that the direction of the acoustic radiation is at a near-right-angle, slightly proximal, to the axis of the catheter. How this happens is not, however, described by the instant applicant. This patent also describes detection of acoustic radiation at the probe by detecting its interaction with an optical signal (e.g., using a laser beam) that is also introduced to the probe tip. A single fiber may run along the catheter and be used, apparently selectively, for conveying ultrasound generating laser light and for detecting acoustic radiation, by using a selectively reflecting surface that passes ultrasound generating radiation and reflects ultrasound detecting radiation. Acoustic interaction between ambient ultrasound waves and sensing light is with a transparent interposing medium between the fiber and the reflector. This patent apparently does not suggest using a same fiber simultaneously for more than one function.

This patent uses a multi-fiber catheter, with each fiber being used to select one angular segment and transmit light and/or ultrasonic energy in a direction generally perpendicular to the catheter axis. Also, a central guidewire is used to guide the catheter. Thus, this design necessarily requires a significantly larger diameter than a catheter utilizing a single fiber.

In addition, the power of the ultrasound generated by this patent is apparently constrained by several fundamental loss processes: (a) most of the powering laser light is apparently lost by reflection from the metallic target, some into surrounding tissue (with an added potential health hazard), and (b) most of the resulting ultrasound is apparently dissipated within the construction of the catheter. The latter effect reduces the effectiveness of the system both in the introduction of uncontrolled ultrasonic signals that introduce large background interference that severely compromises the performance of the device as well as in a significant reduction in the available power . In addition, unwanted power is apparently also absorbed by the surrounding tissue.

## SUMMARY OF THE INVENTION

The invention relates to an ultrasonic generator as defined in claim 1. Preferred embodiments are disclosed in the dependent claims.

## BRIEF DESCRIPTION OF THE FIGURES

Particular embodiments of the invention will be described with reference to the following description of exemplary embodiments in conjunction with the figures, wherein identical structures, elements or parts which appear in more than one figure are preferably labeled with a same or similar number in all the figures in which they appear, in which:

Fig. 1 is a schematic illustration of an ultrasound generating optical fiber, in accordance with an exemplary embodiment of the invention;

Fig. 2A is a schematic illustration of an ultrasound generating optical fiber in accordance with an alternative embodiment of the invention;

Fig. 2B illustrates the absorption of energy in the embodiment of Fig. 2A as modified by reflection, in accordance with an exemplary embodiment of the invention;

Fig. 2C illustrates the absorption of energy in an exponential absorber, in accordance with an alternative exemplary embodiment of the invention;

Fig. 2D illustrates the absorption of energy in a discrete-step absorber, in accordance with an alternative exemplary embodiment of the invention;

Figs. 3A and 3B illustrate the effect of using two side-by-side optical fibers on the resulting acoustic field pattern, in accordance with an exemplary embodiment of the invention;

Fig. 4 illustrates a single optical fiber with multiple light absorbing areas, in accordance with an exemplary embodiment of the invention;

Fig. 5 illustrates an optical ultrasonic system, in accordance with an exemplary embodiment of the invention;

Fig. 6 illustrates the use of a fiber-optic ultrasound source as a guidewire, in accordance with an exemplary embodiment of the invention;

Fig. 7 illustrates the use of a fiber-optic ultrasound source for ultrasonically marking an invasive tool, in accordance with an exemplary embodiment of the invention;

Fig. 8 illustrates a multi-element probe, in accordance with an exemplary embodiment of the invention; and

Fig. 9 is a graph illustrating experimental results of a device constructed in accordance with an exemplary embodiment of the invention.

## DETAILED DESCRIPTION OF EXEMPLARY EMBODIMENTS

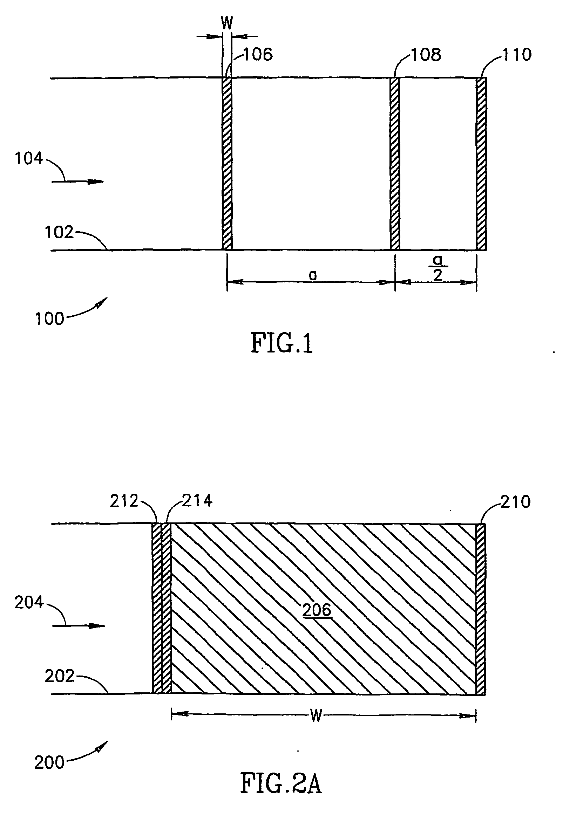

Fig. 1 is a schematic illustration of an ultrasound generating optical fiber 100, in accordance with an exemplary embodiment of the invention. Fiber 100 includes a body 102 through which a pulse (or train of pulses, or another waveform such as a saw-tooth or Gaussian form) of electro-magnetic radiation 104 (indicated by an arrow), for example infrared, ultraviolet or visible light, propagates. At least some of the illumination is absorbed by an absorber 106, thereby heating it and causing it to expand abruptly and emit an ultrasonic wave. This wave is typically a multi-spectral wave. As explained in the following, however, the spectrum and/or direction of the wave may be manipulated.

Potential advantages of using guided-volumetric absorption are:

(a) The generating radiation can be guided through the absorption process and is thereby confined laterally. Lateral spreading of the generating wave through the absorption process as would occur in unguided situations where the beam diffracts and expands, can generally be prevented. The radiation power density is therefore diminished only due to the absorption process and not as a result of beam-spreading;

(b) The absorption can be spread over a greater depth of the target and can therefore generate a more controlled ultrasonic wave; and/or

(c) The use of volume absorption allows for potentially better control of the resulting acoustic waveform, for example by variation in the degree of absorption within the absorbing region.

The ultrasonic wave generated in the absorbing region is essentially the shock wave generated by thermal shock due to the abrupt heating of the absorbing medium. The characteristics of the acoustic signals generated using this thermo-elastic regime possibly derive primarily from the temporal characteristics of the deposited electro-magnetic energy and/or from the geometrical form of the heat deposition the heat-dissipation properties of the surrounding medium. For simplicity, various effects, such as the convection and radiation of heat away from the heated region and the direct coupling of the acoustic and electro-magnetic phenomenon, are neglected. Also for simplicity, only the initial acoustic signal, before it is distorted by traveling through the surrounding medium, is considered, and only the contribution due to the linear response of the material is included. It should be clear that none of these assumptions and/or limitations are critical for actual operation of the invention and they are provided only for simplifying the presentation and for simplified initial calculation.

Under these assumptions the displacement of the generated ultrasound can be represented as:

$$u_k(\mathbf{X}, t) = \alpha_T (3\lambda + 2\mu) \int_V \Theta(\xi, t) \delta_{ij} * G_{ki,j}(\xi, t; \mathbf{X}, 0) \, dV \qquad (1)$$

Where

$u_k(X, t)$      is the ultrasonic displacement in the three orientations, k.

$\alpha_T$      is the linear thermal expansion coefficient of the material

$(3\lambda + 2\mu)$      are the Lamé constants of the material

$\Theta(\xi, t)$      is the instantaneous heat distribution across the heated region

$\delta_{ij}$      is the Kroneker delta function

$*$      denotes convolution in time

$G_{ki,j}(\xi, t; X, 0)$      is the derivative of the Green's function in the j direction

and the integration is performed over the entire heated region. As will be described below, the heated region may be non-uniform or discrete. Alternatively or additionally, for example as described below, even a uniform region can be heated in a non-uniform manner, for example by using wavelength addressing to selectively address different parts of an absorbing region with different energy levels.

The frequency response of the absorber includes various spectral components, as described below, for simplified cases. In a practical implementation, the spectral components may be somewhat different, however, the following discussion may be used as an aid in defining the number and other properties of absorbing areas, in accordance with exemplary embodiments of the invention.

The leading-edge of the Green's functions for displacements is characterized by an abrupt step singularity (Pekeris in Proc. Acad. Sci., 41, pp. 469-480 and pp. 629-639, 1955), causing the leading edge of ultrasonic signal reflect the temporal distribution of the deposited electro-magnetic pulse.

Taking a typical laser pulse with a rise-time on the order of 10 nanoseconds, and, for example, a glass material (for body 102) with a relatively poor heat conduction, the thermal shock, and the resulting acoustic disturbance corresponds almost entirely to the laser-pulse transients, and the initial acoustic wave comprises of the frequency spectrum resulting from a transient excitation of 10 ns. This is a broad-band excitation with a center-frequency on the order of 30 MHz.

The temporal width of the longitudinal component of the ultrasound, as observed in the Green's functions, is on the order of less than 0.01 r/c, where c is the ultrasonic velocity and r the distance of the source from the observation point. For example, if a Gaussian laser pulse of 10 ns width is used for the generation in glass at a distance of 1 mm, the leading edge of the ultrasonic pulse would be on the order of 5 ns. Similarly, for this distance, the contribution of the width of Greens's function is approximately 0.01x1 mm/6,000 m/s = 1.7 ns, so the pulse width for a point-source generator is on the order of the electro-magnetic pulse width. It is expected that a bi-polar pulse be generated, the contribution is in the form of the derivative of Green's function.

Taking into account the volume of the generator, the temporal shape of the initial ultrasonic pulse may be characterized by the convolution of the electro-magnetic pulse shape and the geometry of the heat source, either one of which may be controlled and/or designed, in accordance with exemplary embodiments of the invention. Considering a square source cross-section of width of 1 mm, one obtains an ultrasonic wave with two main features - the bipolar pulse ensuing from the edges of the illuminated region, with a width commensurate with that of the electro-magnetic pulse, and a residual ultrasound pulse corresponding to the width of illuminated region (this is due to any asymmetry in the bi-polar Green's function derivative). Consequently two major frequency components are observed - a pulse with a time width comparable to the width of the electro-magnetic pulse, and a central component with a wavelength comparable to the width of the heated volume.

For example, a single region of width w is expected to generate ultrasound with a central frequency for which w corresponds to half an acoustic wavelength. For example, in glass, with acoustic velocity of nearly 6,000m/s, a uniformly illuminated absorbing region of width *w=½×6,000/30 MHz = 0.1 mm* (and odd multiples thereof) reinforces the first wavefront ensuing from the first thermal shock front with the wavefront, of opposite sign, ensuing from the opposite edge of the thermal shock font. In another example, for a glass target and 1 mm illumination, this corresponds to a central frequency of $f=2c/w = 2x\ 6,000m/s/1mm = 12MHz$. The relative strength of this component as compared to that ensuing from the edges depends, *inter alia*, on the thermal gradient at the boundary of the thermal source: the sharper this gradient the stronger the contribution of the edge component in the signal; conversely as the thermal boundary becomes more gradual or blurred, the lower frequency contribution of the width of the source increases in importance. As discussed below, the attenuation of the generating electromagnetic radiation as it travels along the absorbing region, introduces a gradual boundary to the region and effectively strengthens the relative low-frequency component generated. Maintaining the absorbers short (small values of w), as drawn in Fig. 1, enhances the relative strength of the higher frequency components in the generated ultrasound.

In an exemplary embodiment of the invention, at least one additional absorbing region 108 is provided distal of absorber 106 to absorb at least some of the light (if any) that is not absorbed by absorber 106. In an exemplary embodiment of the invention, the distance between the absorbers, a, and their extent in the axial direction, w, serve to design the desired ultrasonic characteristics of the resulting waves as discussed below.

In an exemplary embodiment of the invention, a low frequency component is generated by increasing the length of the absorbing region in the fiber and/or using a series of suitably spaced heating regions. For example, to generate a 600KHz acoustic signal, a series of regions of length $w=\lambda/2$ and a similar spacing can be used. In a glass waveguide, $\lambda/2= \frac{1}{2}c/f = \frac{1}{2} \times 6,000m/s/600KHz \rightarrow$ regions 5 mm in length may be used. In general, the spatial distribution of heated volume is related to a Fourier Transform of the resulting spectrum, depending on the envelope of the illumination. Increasing the number of absorbers narrows the width in the Fourier plane and the resulting spectrum of the signal. As the boundaries of the absorbing regions are made more gradual the high-frequency components are reduced. Similarly, introducing a monotonically changing region spacing and length results in a time-variable spectrum or chirp signal. Consequently, to reinforce a particular frequency component in the generated acoustic wave, the *spacing*, a, between the absorbers has to correspond to the acoustic wavelength of that component. This is shown schematically in Fig. 1 where absorbers 106 and 108 are spaced by $\lambda$- the acoustic wavelength.

If thin absorbing volumes are used, they may each generate a very high intrinsic acoustic frequency, as determined by their geometrical width and the rise-time of the generating electromagnetic pulse. For example, for a 10 ns rise-time pulse, and an absorber that is narrower than say 0.01 mm, can give rise to ultrasonic components at 300MHz or more. The distance between the absorbing regions determines a lower frequency, with a generally lower power. If, as in Fig. 2A the absorbing region is wide, the lower frequency component is stronger. Optionally, the lower frequency component is made to dominate the waveform by providing a gradual change of absorption in at least part of the absorbing region boundary. In this manner the edge effects are subdued and the volumetric effects over the extent of the absorbing region, dominate. In an exemplary embodiment of the invention, the boundary area may comprise a linear increase in optical density over a length that is, for example, 1%, 5%, 10%, 20% or any smaller, intermediate or greater percentage of the length of the absorbing region.

Optionally, a reflector 110 is provided distal of absorber 108, for example, at a tip of fiber 100. This reflector returns light that passed absorbers 106 and 108, to be absorbed by the absorbers. Alternatively, absorber 108 is a total absorber of all the light and reflector 110 can be omitted. In an exemplary embodiment of the invention, to reinforce a particular frequency component in the generated acoustic wave, the *distance* between the last absorber 108 and the reflector 110 should correspond to *half* the acoustic wavelength of that component. This is shown schematically in Fig. 1 where absorber 106 and 108 are spaced by and acoustic wavelength, $a=\lambda$, while the distance between absorber 108 and the reflector 110 is half that value, $a/2=\lambda/2$.

In the reflector embodiment, the acoustic signal will have two sets of super-imposed components, two due to the absorption of electro-magnetic wave on the forward travel, and two due to the backward travel of the electro-magnetic after reflection from the tip of the waveguide which is fully reflective; as the speed of electro-magnetic radiation is very much larger than that of the ultrasound, the two sets of acoustic waveforms super-impose, optionally compensating for the decay of the incident electro-magnetic power with distance. The second absorption region receives reduced incident power due to the absorption in the first region, but on the return pass the situation is reversed. As the absorbing regions are suitably spaced, and their degree of absorption can be controlled, the relative intensities of the four components in this case can be designed to suit the application. One potential advantage of this approach is the ability to generate a unique acoustic waveform that can be readily identified by its specific characteristics in the system. Another potential advantage of this approach is the ability to generate a more uniform acoustic waveform as compared to other arrangements where the acousto-optic interaction is confined to a small region or a boundary layer.

In an exemplary embodiment of the invention, the absorbing regions are dichroic, permitting the transfer of a second electro-magnetic wavelength. As noted below for various embodiments, this allows the size, number, location and/or intensity of the absorbing regions to be controlled in real-time or prior to use of the system, by having participating absorbing volumes being selected by wavelength. The combination of source parameters including the dimensions of the absorbing regions, the degree of absorption and the distribution of the absorption profile within the absorbing region, the separation of the regions and the intensity and rise-time of the generating radiation pulse or pulses, controls the characteristics of the ensuing ultrasonic waveforms. It is thereby possible, by judicious choice of the above parameters to control the directionality and direction, the frequency content, the overall envelope and the intensity of the generated signal, by design and/or by selective manipulation of various illumination parameters.

As noted above, the relative absorption properties of absorbers 106 and 108 and/or the reflective properties of the mirror may be used to achieve a desired spatial absorption profile in the fiber. Optionally, for the same or a different purpose, at least one of the absorbers does not cover the entire cross-section of the fiber, to allow a predetermined portion of the light to pass and possibly be absorbed and/or reflected at a later time. Alternatively, the absorbing area is polarization dependent, for example itself acting as an absorption polarizer, so that it only absorbs one component of light polarization. An absorber may be one or more of dichroic, polarization dependent and spatially varying in the cross-sectional direction.

In an exemplary embodiment of the invention, the absorbing regions are defined inside the fiber, for example, by doping a material (e.g., glass) of which the fiber is made or by introducing deliberate damage, applying stress or otherwise modifying the material continuum or uniformity. Alternatively or additionally, the fiber is cut and spliced with an absorbing fiber section (e.g., a colored or polarizing fiber) and/or an absorbing material section, for example a plate colored material or a linear polarizer, which are optionally coated with a cladding. For example, for near-IR radiation doping with and absorber such as CuSO4 produces the desired absorption region. This may be introduced into the fiber by splicing an undoped fiber with section of a similar fiber with such doping.

For clarity, cladding of fiber 100 is not shown in Fig. 1. In some embodiments of the invention, absorption is provided in the cladding, for example, by replacing a section of the cladding with an absorbing material. Alternatively or additionally, the refractive index of the cladding is modified to allow some light to leak out and be absorbed by an absorber outside of the fiber. A potential advantage of this type of mechanism is that some patterns of absorbing regions may be easier to manufacture outside of a fiber. Such a change in the cladding may, however, cause dispersion problems in the fiber, which are expected to be insignificant in many cases.

Fig. 2A is a schematic illustration of an ultrasound generating optical fiber 200 having a body 202, in accordance with an alternative embodiment of the invention. Unlike fiber 100 (Fig. 1), fiber 200 utilizes an extended absorber 206 that has a length close to $\lambda/2$ (half the desired central acoustic wavelength) to maximize the generation of the desired acoustic frequency component. Other lengths may be used as well and depend, inter alia, on the existence of a nearby fiber end and/or a reflector. In an exemplary embodiment of the invention, a light pulse indicated by an arrow 204 is absorbed along absorber 206. Optionally, a mirror 210 is provided to reflect unabsorbed light back along absorber 206. The length of the absorber here can approach $\lambda/2$. Using the same parameters as before, the length of the *absorber* would now be some $w=\frac{1}{2}\times 6,000/600KHz = 5\ mm$ for generating a strong 600KHz component. Note that, although Figure 2A shows region 206 at the tip of the fiber, it can equally well be located at a distance from the fiber tip.

In an exemplary embodiment of the invention, for example, in fiber 200 or in fiber 100, multiple reflections through the absorbing regions are provided to make the generating region more uniformly excited. In one example, the arriving wave 204 is passed through a polarized beam splitter 212 and then through a quarter wavelength plate 214. In operation, incident light in one polarization, is transmitted through beam splitter 212, rotated 45° by wave plate 214 to form circularly polarized light and on reflection from the mirror at the end of the waveguide, rotated again to become incident on splitter 212 in an orthogonal polarization state. Therefore the incident beam traverses the absorbing region twice before it is rotated to the original polarization and leaves the volume. In some embodiments of the invention, the fiber itself is made with special polarization properties, for example, being polarization preserving.

This reflection method reduces somewhat the non-uniformity found in relatively large absorbing regions due to the decay of the illumination as it propagated. When the illumination is reflected to travel again through the absorbing region the absorbed intensity on the forward pass decays in the forward direction while the absorption on the reverse pass decays in the opposite attitude thereby forming a more uniform overall acoustic energy source. This is useful, for example, for lower-frequency generation where the length of the absorbing region corresponds to the dominant acoustic wavelength generated. Lower frequency US may be used for ablation of plaque and unwanted tissue where the incident energy has to be designed to generate sufficient cavitations or mechanical resonance of the target; typically lower frequencies are used for this purpose.

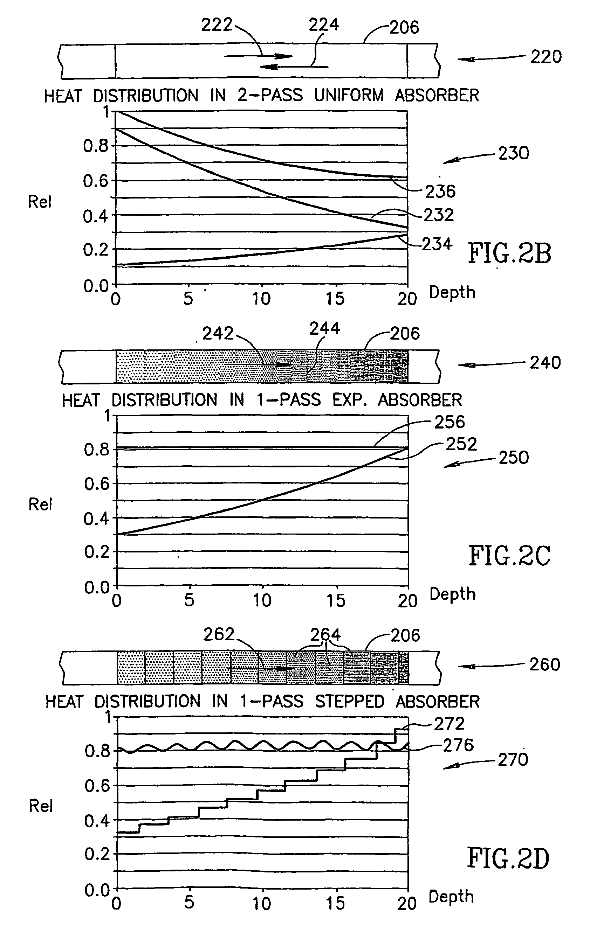

Fig. 2B shows the effect of reflection on the uniformity of energy absorption. Reference 220 shows forward and backward propagating light 222 and 224 (in a two pass example). Reference 230 is a graph showing, super imposed, relative forward radiation absorption 232, relative backwards radiation absorption 234 and total radiation absorption 236. The total absorption corresponds to the actual intensity of emitted ultrasonic radiation.

Optionally, the density of absorber 206 varies in a manner that takes into account the reduction wave amplitude and/or reflection, so that thermal heating is uniform or has a different desirable form. For example, to generate a side-looking component at an off-perpendicular direction, a decaying distribution can be used. Another example is a sinusoidal absorption characteristic (whether strictly or piece-wise sinusoidal) for reinforcing the generation of a certain acoustic frequency.

Fig. 2C illustrates the absorption of energy in an absorber having an exponential absorption coefficient, in accordance with an alternative exemplary embodiment of the invention. Reference 240 shows absorber 206 within a waveguide with an exponentially graded absorption 244, for absorbing forward traveling light 242. A graph 250, shows an absorption density 252 increasing exponentially, so that when interacts with the actual beam, the result is uniform absorption of energy 256 along absorbing region 206 and therefore a relatively uniform energy distribution.

The uniformly varying absorption profile of Fig. 2C may be relatively difficult to manufacture. In an exemplary embodiment of the invention, the exponential profile is approximated by a discrete series of individual absorbers, each with a possibly uniform absorption profile and adjacent or spaced apart. Fig. 2D illustrates the absorption of energy in a discrete-step absorber, in accordance with an alternative exemplary embodiment of the invention. Absorber 206 comprises a plurality of absorbers 264, each with a different absorption coefficient, for example, with an exponential increasing coefficient between the absorbers. Although the absorbers are shown in contact with each other, they may be spaced, for example by an absorption-free waveguide portion. A graph 270 shows a piece-wise approximation to the exponential absorption profile 272, with a resultant energy deposition 276 that is substantially spatially uniform, e.g., with small variations.

In an exemplary embodiment of the invention, only a small number, such as 2, 3 or 4 absorbers are provided, for example. Reflectors may be provided, of course in the embodiments of Figs. 2C and 2D. Alternatively, a larger number of absorbers, such as 10, 20 or any intermediate smaller or larger number, may be provided.

The apparatus described above can be used to generate ultrasound for many different applications, of which several examples are: ultrasonic treatment; ultrasonic ablation; indirect heating using ultrasound; sonophoresis; ultrasonic monitoring of various parameters, such as thickness or depth; ultrasonic characterization of a target material and/or for imaging; and photo-acoustic imaging or characterization of a target material. Optionally, as described below, a plurality of different ultrasound sources are provided.

In an exemplary embodiment of the invention, the light source is laser light, optionally, from a wavelength tunable laser. We note that the choice of laser light is a matter of convenience only and since, in some embodiments, there is no requirement of the coherence of the source, a flash lamp or other optically gated light sources are viable alternative sources.

In an exemplary embodiment of the invention, the absorbers are wavelength selective. For example, laser treatment light passes through substantially unaffected while laser for ultrasound generation is absorbed. Alternatively to treatment, the transparency to some wavelengths may be used for optical operations, such as providing light illumination and/or detecting light.

In an exemplary embodiment of the invention, ultrasound detection uses acoustoelectric or piezoelectric transducers (not shown) mounted near the tip of fiber 100. Alternatively, optical means are used to detect acoustic waves. In an exemplary embodiment of the invention, acoustic signals are detected using an opto-acoustic interaction with an acoustically sensitive optical material provided in the fiber. In an exemplary embodiment of the invention, a detection beam travels through the fiber and passes through an acoustic sensitive material incorporated in or adjacent to a reflector at the tip of the fiber. The acoustically sensitive material may be the same material used for ultrasound generation or it may be separate. In an exemplary embodiment of the invention, a birefringent material (not shown) is provided near reflector 110 as a detector so that a reference beam of light having a wavelength not absorbed by the absorbers, is affected by changes in the birefringence that are dependent on stress in the fiber (e.g., stress from externally impinging acoustic waves). Alternatively, the fiber as a whole may be birefringent whether by design or inadvertently by the production processes. Alternatively or additionally, other optical detection methods may be used, to demodulate the effect on the sensing wavelength or wavelengths, for example, such as Fabry Perot resonator, Polarimetric measurements, Interferometry of various types (e.g., homodyne, heterodyne, speckle, Fucou, Sagnac, holographic), Bragg-grating spectral analysis and/or other optical demodulating methods known to the art. The demodulation can be implemented entirely within the fiber, or optionally, some or all of the demodulation means can be situated external to the fiber, for example, in a controller external to the fiber (e.g., controller 506 described below).

Alternatively or additionally, the boundaries of the absorbing regions act as partial reflectors that are displaced by the impinging acoustic waves. This displacement generates an inference pattern in the detection light, which may be read out, for example, by the controller using optical demodulation techniques and/or signal processing methods known in the art. Alternatively or additionally, reflector 110 may be moved by the acoustic waves, to modulate the sensing wavelength, for example by generation of an interference pattern. In an exemplary embodiment of the invention, the displacement and/or compression of the tip of the fiber which is immersed in an acoustic field is detected by its effect on a detection wave that is reflected from the fiber tip. The reference wave used for detection may pass through the absorbers (completely or partially) or it may be reflected before the absorbers, for example, by beam splitter 212 (e.g., having a different polarization), thus allowing a same wavelength to be used for generation and detection. Detection may be provided at one or more other points along the fiber in addition to or instead of the fiber tip. An alternative to a reflecting surface is a reflecting grating or phase array or scattering array that may be impressed into the fiber material by a variety of methods, including, for example, laser etching.

The shape, location and/or activation of the absorbing regions in one or more nearby fibers can be used to achieve various effects, especially, beam aiming, enhancement of a particular spectral component within the generated ultrasound and/or otherwise selecting a frequency spectrum.

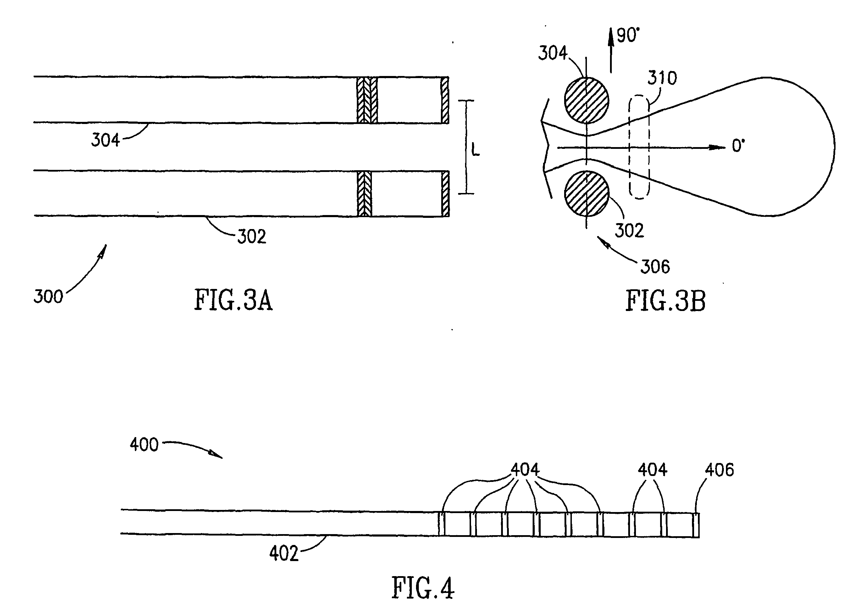

Figs. 3A and 3B illustrate the effect of using two side-by-side optical fibers on the resulting acoustic field pattern, in accordance with an exemplary embodiment of the invention. Such multiple fibers are driven as a phased array, in some embodiments of the invention. In other embodiments of the invention, the fibers are driven as a mono-pulse system as explained below. Fig. 3A (reference 300) shows a side-view of two fibers 302 and 304, for example of the type shown in Fig. 1 or in Fig. 2. The two fibers are separated by a distance L, which may be constant or vary along the ultrasound emitting areas. Fig. 3B (reference 306) is a front view of the two fibers. In the example shown, the two fibers are driven in phase, so that the main lobes of the generated acoustic waves are directed along the normal to the centerline of the fiber array at 0° and 180°. Only the 0° lobe is shown, for clarity. Other relative phases effect other beam directions. In an exemplary embodiment of the invention, one of the lobes is blocked, for example, by an absorbing material 310 (depicted in the figure as a block of the lobe at 0°), so that essentially one, directional beam ensues from such a two-fiber probe assembly. Alternatively or additionally, part of a lobe may be blocked. Alternatively or additionally, a plurality of fibers are arranged in an array, for example, a two dimensional array, such as a hexagon or a linear array, allowing a finer control over the beam direction. Optionally, the different fibers of the array are driven with controlled light intensities to effect simultaneous phase and/or amplitude control.

In an exemplary embodiment of the invention, the multiple fibers are used for phased-array type or mono-pulse type detection of acoustic fields. In mono-pulse detection, the field at each fiber is detected separately and/or each source is activated separately and then the results are processed together. In the two-sensor example of Fig. 3, this amounts to three measurements - one with the first fiber only, one with the second fiber only, and one with both fibers activated simultaneously. As the ultrasonic beam patterns differ for each of these measurements (e.g., due to their covering different areas/angles), a target reflects at different intensities in each measurement. The differences in the measured intensities be can related back to obtain information on the spatial location of the target, for example using methods well known in the art of radar.

In another example, ultrasonic beam directivity is obtained by introducing fibers with preferred ultrasound emission directions, for example using absorbing cladding covering most of the angular range of each fiber except for a specific designated emitting angular window. Active sweeping may also be obtained, for example, by changing the phase difference between fibers in a fiber pair. Various directionality properties may also be achieved by varying the relative intensity of the irradiation of the two fibers.

Fig. 4 illustrates a single optical fiber 400 having a body 402 with multiple light absorbing areas 404 (e.g., 2, 3, 4, 5, 6 or more regions) and an optional tip region and/or reflector 406, in accordance with an exemplary embodiment of the invention. In an exemplary embodiment of the invention, the absorbing areas are selective to different wavelengths. Thus, the location of ultrasound emission is dependent on the wavelength used. Alternatively or additionally, multiple absorbing areas are excited, to provide relatively long ultrasonic sources (e.g., for heat treatment or for generating low frequencies). Alternatively or additionally, multiple wavelengths are used simultaneously, possibly at different pulse rates and/or relative phases. Thus, a plurality of ultrasound sources can be created at desired relative phases and pulse rates, allowing various interactions between the sources to be provided. Alternatively or additionally, the signals from these sources can be distinguished during detection, possibly using a single detector, for example, based on different pulse repetition rates, pulse envelopes and/or frequencies of the different sources.

In an exemplary embodiment of the invention, by selecting the location of excitation, the direction of a beam, relative to the axis of the fibers, in a multiple-fiber arrangement can be controlled. Alternatively or additionally, each location 404 is a polarization dependent absorber (e.g., a polarizer) and the ultrasonic source location is selected by changing the polarization alternatively or additionally to changing the wavelength. For example, if two absorbers with perpendicular polarization axes are provided, sending light with the polarization of the first absorber, will allow the light to pass the first absorber and be absorbed by the second. The absorbers may also be wavelength dependent and/or have non-perpendicular polarization axes.

In some embodiments of the invention, various "addressing" schemes may be used, in which certain pulses are directed to certain absorbing regions, based on previous pulses. For example, if photo-activated absorbers are provided, one wavelength (e.g., ultraviolet) can be used to "activate" an absorber by changing its absorption characteristics, and a second (e.g., high-energy pulse) will then be absorbed and used to generate the ultrasound. For example the material sold as "Photogray", used in sunlight accommodating eye-glasses, can be used.

In another example, the absorber is wavelength-dependent along its cross-section, exhibiting a different behavior on different parts of the cross-section; again, this may be used for beam forming. Alternatively or additionally, some of the cross-section is transparent to allow light to pass on along the fiber. Alternatively or additionally, for example in larger, multimode fibers some of the cross-section absorbs one wavelength of light and some a different wavelength of light. The regions may have different lengths and/or they may overlap in cross-section. It should be noted that providing absorption of different wavelengths at different sectors of a cross-section is functionally equivalent, in some applications, to providing multiple fibers.

In a more generalized manner, the interaction between multiple sources can be analyzed with respect to two major axes, the radial and the axial.

In the radial direction the presence of a second ultrasonic source and the resulting acoustic field corresponds to that of a dipole axial source. A separation, *a*, between the sources (as shown in Fig. 1) determines the directionality of the different frequency components of the combined generator. It should be appreciated that *a* is related to a phase difference between the two sources, which may also depend on frequency and on an imposed phase difference in driving the sources. As noted above, phasing the source activation in real-time allows for a real-time variation in the parameters of the acoustic beam, including for example an angular sweeping of the beam. The directionality of the combined elements is often strongly frequency dependent and therefore, since the sources are typically broad-band, a spectral analysis of the detected components relates to different radial directions of the system. In one embodiment of the invention, this information can be used to generate an image with its circumferential pixel elements being detected at different frequencies. Alternatively, frequency division of functions can be effected. For example, for a simple source, low frequencies (e.g., for treatment) propagate perpendicular to the axis of the sensor array, while high frequencies (e.g., for imaging and/or treatment) propagate at an angle to this direction. For example, as the frequencies increase such that the separation a approaches half an acoustic wavelength, the main ultrasound beam will be directed further and further off this direction approaching, at the limit, the direction along the axis of the array.

Along the axial direction the separation of the individual absorbing regions carries a different significance - source separations in multiples of an acoustic wavelength will reinforce, while others will destruct; consequently, depending on the number of sources, a frequency and/or spatially narrower band signal is generated at certain predetermined frequencies. As should be appreciated, such a signal can also be steered in the azimuth direction, in accordance with exemplary embodiments of the invention.

In an exemplary embodiment of the invention, the physical and/or geometrical characteristics of the absorbing regions are designed mathematically, e.g., based on wave generation and propagation equations. Alternatively or additionally they are designed iteratively, using real and/or a simulated model.

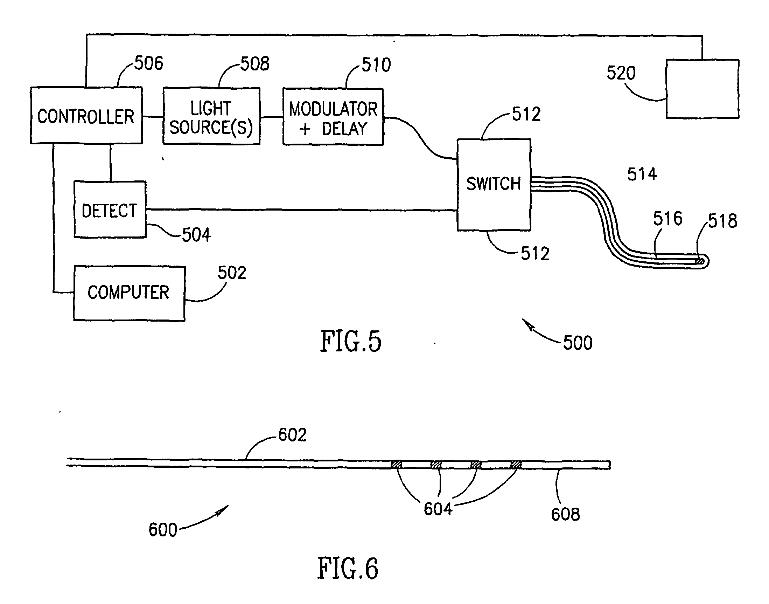

Fig. 5 illustrates an optical ultrasonic system 500, in accordance with an exemplary embodiment of the invention. In an exemplary embodiment of the invention, a probe 514 comprises at least one optical fiber 516, such as those described above, that includes an ultrasound generating and/or detecting tip 518. Light for generation of ultrasound and/or outputting a beam of light at tip 518 is provided by one or more light sources 508, for example a laser source and/or a flash lamp. In the case of a flash lamp, a filter with one or more spectral pass regions may be provided, for generating a desired spectrum.

The light from the sources is then optionally modulated (e.g., to provide a pulsed source or a different envelope, such as saw-tooth, sinusoidal or one that relates to the desired acoustic waveform) by a modulator and delay source 510. The delay or pulsing phase difference between different light beams may be used, for example, to control a beam direction. In some embodiments, the source is self-modulated (e.g., a pulsed laser).

It should be noted that in many embodiments of the invention a probe 514 can comprise only a single fiber, with a relatively small diameter. Optionally, this fiber is coated with various materials, such as anti-coagulants and bio-compatible polymers. Alternatively or additionally, a hollow waveguide is used.

In some embodiments of the invention, multiple fibers and/or multiple sources are used. In these a coupler or switch 512 may be provided for coupling the light to probe 514 and couple detection light from probe 514 to a detector 504 (if necessary). The generation and detection of light may be controlled, for example, by a controller 506. Optionally, a computer (e.g., a microcontroller) 502 is provided, for example, for a user interface and/or for storing recorded signals, images and/or other data.

An external device 520, for example, an imager, a sound source and/or a treatment device may be controlled by controller 506. In an exemplary embodiment of the invention, the imager is used for reconstructing an image based on acoustic radiation provided by probe 514. Such reconstruction may be, for example, based on detection of transmission and/or reflection radiation, as known in the art. Alternatively or additionally, the imager is used to detect the position of probe 514. An external ultrasound source may be used instead of or in addition to a sound source in probe 514, with probe 514 being used for detection of the sound and providing an image or other information. A separate treatment device may be controlled by the computer to treat about probe 514, for example, to remain aimed at probe 514 and/or using information or an image from probe 514. Alternatively, manual coordination may be used. The system may also be employed for photo-acoustic imaging where an independent sensor (possibly utilizing the same or a similar optical fiber) maps the temperature of the object under test as the probe tip is scanned through various positions.

Depending on the exact implementation, one or more of the following features may be provided in system 500:

(a) Generation of ultrasonic waves for heating tissue, for example, using lower frequency ultrasound and/or ultrasound generated along a significant length of probe 514.

(b) Generation of ultrasonic waves for fragmenting plaque, stones or other unwanted tissue. Again, lower frequency ultrasound, possibly in a forward direction, may be used. Suitable frequencies and power levels are known in the art.

(c) Generation of ultrasonic waves for imaging, e.g., narrow bandwidth or wide bandwidth, of various frequencies.

(d) Generation of a specialized waveform of ultrasonic waves, for example a train of pulses at well-controlled intervals, or a chirp. For example, a series of absorbing regions are spatially spaced in order to generate the desired temporal behavior of the ultrasonic wave. For example a train of ultrasonic pulses is obtained by a sequence of relatively thin absorbers. The thickness of the absorbers corresponds to the width of each pulse and their separation corresponds to the spacing between the pulses. Using monotonically varying separations and absorber lengths can generate a chirp waveform.

(e) Provision of a forward- or side-looking (e.g., using an angled mirror in or adjacent the fiber) laser light.

(f) Detection of acoustic radiation.

(g) Usage of fiber 516 as a different type of detector for a variety of parameters known in the art of optical fiber sensors, for example a temperature sensor, a pressure detector, electric or magnetic field sensor or chemical sensor.

(h) Generation of directional or omni-directional acoustic fields, for example for effecting sonophoresis for enhancing absorption of pharmaceuticals provided near and/or by probe 514.

(i) Generation and detection of ultrasonic waveforms for characterization of the target material or dimensions, for example based on spectral reflection or other methods known in the art of ultrasonic characterization.

(j) Generation of periodic acousto-thermal signals for imaging and characterization of a target in methods known in the art of photo-acoustic imaging and characterization.

Thus, system 500 (optionally in conjunction with an external device 520) can be used for one or more of the following applications: US plaque fragmentation; laser plaque removal and monitoring; artery dimension monitoring; intra-body measurements and imaging (for example using A-mode and/or Doppler); and/or drug delivery enhancement. Probe 514 can be, for example, a catheter or an endoscope. In an exemplary embodiment of the invention, probe 514 includes an inflatable distal portion, for example a balloon, to ensure contact with surrounding tissue and/or to fix the gaze direction of probe 514.

Fig. 6 illustrates the use of a fiber optic ultrasound source 600 as a guidewire, in accordance with an exemplary embodiment of the invention. A guidewire is widely used in coronary procedures and is typically characterized by having a small diameter, and sufficient flexibility to negotiate the bending in the arteries or other ducts it is introduced into. While some energy may be lost at small bending radii, this is generally not a problem as sufficient energy may be provided from outside the body. For this application source 600 is optionally enclosed in a suitable protective jacket. The resulting device may be made of similar dimension as a standard guidewire and handled with the same procedure, with the significant advantage of potentially offering ultrasonic sensing. Such sensing may be used, for example, for viewing branches in blood vessels during navigation and/or for detecting a stenosis area, and/or for measuring a vessel's dimensions. This advantage alleviates the need to alternately introduce different surgical tools to the treated region, as is state of the art: the guidewire serves to mechanically guide the medical treatment devices - such as stent applicators. It can thereby eliminate the need for applying additional imaging and/or diagnostic tools.

In an exemplary embodiment of the invention, guidewire 600 comprises a single (or small number) of fibers 602 having one or more absorbing regions 604 defined along its length. Optionally a tip 608, for example, a flexible tip or a different type of tip as known in the art of guide-wires is provided at a distal end of guidewire 600. Regions 604 may be used for generating ultrasound, for example, to be detected on an external (or another implanted) imager. Alternatively or additionally, regions 604 are used for imaging sideways or forward and/or for detecting distances and/or obstructions. In the case of a guidewire, viewing in A-mode, of a single pixel distal of the guidewire tip may be useful, for example, for detecting forks in vessels, determining a depth of plaque and characterizing its components. Optionally the guidewire is used for measurements for example vessel diameter, wall-thickness and stenosis type and/or thickness, which may be useful, for example, in selecting a suitable stent for implantation.

In an exemplary embodiment of the invention, guidewire 600 is used to carry a stent and/or a PCTA balloon, which may be mounted on the guidewire or conveyed along it.

Another use of the potentially small profile of a fiber optic acoustic source is using a fiber as a marker or beacon, for example, for indicating a tool on an ultrasound image or for showing its future path. In this use, the fiber is typically used as a beacon, for example a point beacon or an elongate (e.g., multi-point) beacon. Alternatively, the vibration of the fiber is used to create a Doppler shift in incident radiation. In an exemplary embodiment of the invention, the wavelength of generated ultrasound is made to match that of the imaging system (e.g., 520 of Fig. 5) so that the beacon is clearly distinguished.

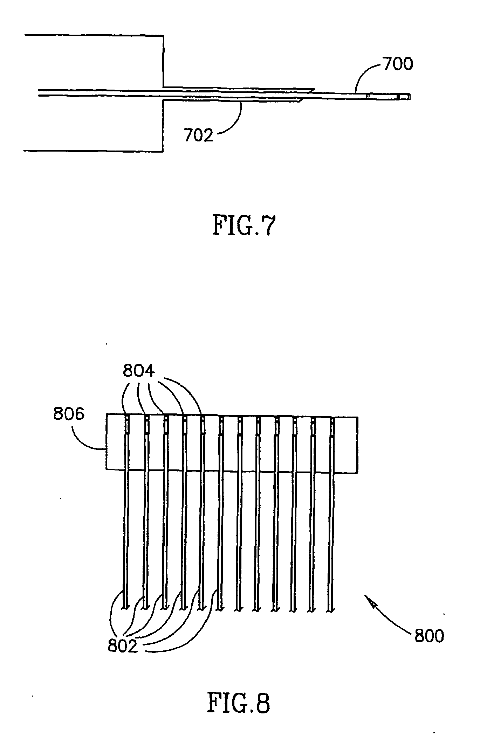

Fig. 7 illustrates the use of a fiber optic ultrasound source 700 for marking an invasive tool 702, in accordance with an exemplary embodiment of the invention. In the figure, the invasive tool is a hypodermic needle and the probe passes through the needle possibly without causing significant obstruction thereof. In some embodiments of the invention, ultrasound source 700 is used for position determination of tool 702 alternatively or additionally to being used for imaging as described above. Alternatively or additionally, source 700 is used as a detector to home in on an acoustic beacon, for example a beacon provided by a different implanted fiber. In principle, as ultrasound can traverse the material of the invasive tools, such as the needle, the fiber ultrasonic source can be completely surrounded by the tool, or, as shown in Figure 7, can be allowed to protrude beyond the tool.

In an exemplary embodiment of the invention, two or more ultrasonic sources are used to better locate the marked tool. If only a point source is used the only indication that can be obtained using a simple detector is the distance to the beacon and the marked tool is known to be located somewhere on a sphere. By providing two or more sources, positioned a known distance apart, the tool can be positioned at the intersection of the tool length with the two spheres scribed by the distances measured to either source. This reduces the ambiguity of the location, in most practical situation, to a conical surface in 3D space. If, for example, the system tracks the relative motion between the imaging system (e.g., detector) and the sources the ambiguity can be reduced further by the generation of a family of such conical section in space that cut each other in a decreasing area. Thus, the condition of "physical" continuous motion of the tool offers an unambiguous solution for the position of the beacon in space. Alternatively, a plurality of detectors or more than two sources may be used. To assist the discrimination between the beacon signal and the standard imaging signal of the imaging system, the signal from the beacon can be designed to produce a specialized waveform which can readily be separated from the imaging signals. For example a train of pulses or a chirp, while essentially at the same frequency as the imaging system, can readily be distinguished from the imaging signals. Alternatively or additionally, a source that generates different frequencies at different points along its length may be used and identified (e.g., utilizing different wavelength selective absorbers with different geometries).

Additional potential advantages of an acoustic-optical transducer in accordance with exemplary embodiments of the present invention, include:

(a) Transferring significant power to a catheter tip.
(b) Reduced diameter probes.
(c) Ability to be used in strong magnetic fields such as MRI fields.
(d) Avoiding grounding problems, especially when the probe is used under field conditions.
(e) Simplicity of construction.
(f) Low cost of the active portion of the system, which can be discarded and replaced after each (or a small number of) surgery procedure.

In some embodiments of the invention, an opto-acoustic transducer as described above is used for a multi-element probe, which may, for example, be used outside the body. Fig. 8 illustrates a multi-element probe 800, in accordance with an exemplary embodiment of the invention. In an exemplary embodiment of the invention, probe 800 comprises a plurality of fibers 802 each with an acoustically active tip 804. The tips are arranged, for example, in a probe body 806. Each fiber may be activated individually. Alternatively or additionally, the fibers are activated in concert, for example as a phased-array. In an exemplary embodiment of the invention, the fibers are powered using a flash lamp, for example, using an electrically controlled LCD to selectively pass light to fibers. Unlike standard piezoelectric transducers, probe 800 does not typically require high voltages (or any voltages) at body 806.

The fibers are typically oriented in a linear array, laid side-by side, each fiber generating in the side-looking configuration. The beam manipulation in the plane of the array vector may then be provided by phasing the generation of each fiber-element. The manipulation of the resulting beam in the perpendicular direction may be effected by the multiple generating/receiving elements in each fiber. In this manner a two-dimensional phased array can be formed. Additionally or alternatively, the fiber sources can be used in the forward-looking configuration. In this option a one- or a two-dimensional array is formed by assembling the fiber tips in a line or a two-dimensional matrix, respectively. In this case the beam is optionally manipulated by phasing the transduction of all the array elements. In both of the examples above, a suitable ultrasonic isolation medium is optionally provided to minimize the cross-talk between adjacent elements.

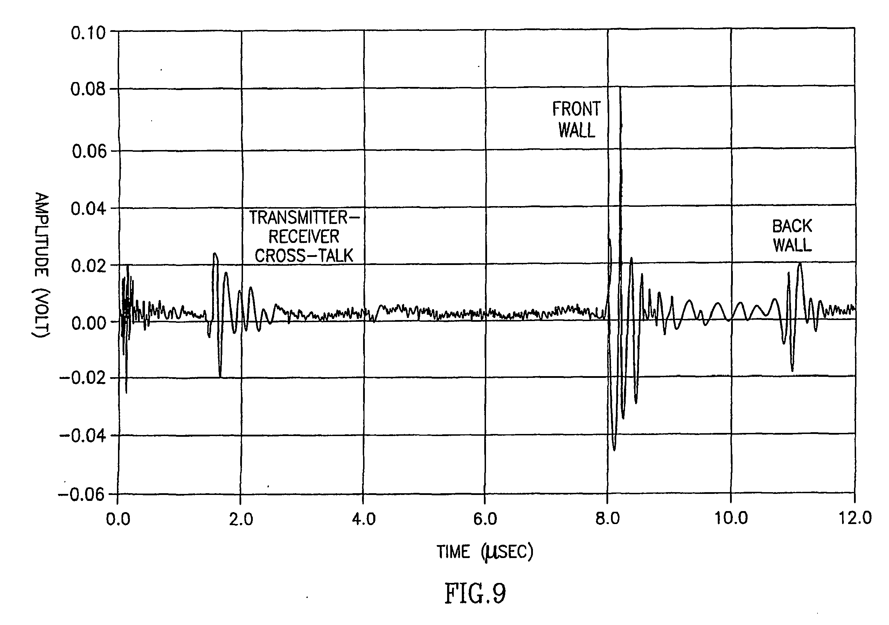

Fig. 9 is a graph illustrating experimental results of a device constructed in accordance with an exemplary embodiment of the invention. This signal was acquired using a single absorbing region transmitter fiber and a polarization-demodulated birefringent fiber receiver. The device is inserted in a lucite tubing filled with saline. The first signal relates to the direct acoustic cross-talk between the transmitter and the receiver. The tubing wall generates acoustic signals from its front- and back-surfaces. Note the reversal of the signal phase at the front wall as expected from a low to high acoustic impedance. For reasons of convenience, a liquid target was used instead of a solid target for generating the ultrasound. However, as noted above, this may be provided in some embodiments of the invention. Generation is effected with a laser pulse of $1\mu J$, 10ns rise time at 1,064nm. Detection with a 532nm laser, and approximate power of 5mW. The generation and detection fibers, both multimode, are positioned about 1mm apart and some 5mm from the wall of the tubing in a side-looking arrangement. Tube wall is approx. 2mm thick. The frequency of the generated ultrasound is approx. 3MHz as expected from the generating region used: a gradual boundary liquid region mounted onto the fiber, approximately 0.8 mm wide.

While the above description focused on optical fibers, other waveguides may be used, for example hollow, lens-series or mirror waveguides for long wavelength infra red radiation. One possible reason for using such waveguides is that a same waveguide is used for generating acoustic energy and for conveying electromagnetic radiation (e.g., RF radiation). Alternatively or additionally, the illuminating electro-magnetic radiation may be RF radiation, with the waveguide being of a suitable type.

The present invention has been described using non-limiting detailed descriptions of embodiments thereof that are provided by way of example and are not intended to limit the scope of the invention. It should be understood that features and/or steps described with respect to one embodiment may be used with other embodiments and that not all embodiments of the invention have all of the features and/or steps shown in a particular figure or described with respect to one of the embodiments. Variations of embodiments described will occur to persons of the art. In addition, some embodiments are described as method or as apparatus, the scope of the invention includes apparatus, for example, firmware, hardware and/or software for carrying out the method and/or methods for using the apparatus, as well as computer readable media and/or communication signals on which such software is stored.

It is noted that some of the above described embodiments may describe a best mode contemplated by the inventors and therefore include structure, acts or details of structures and acts that may not be essential to the invention and which are described as examples. Structure and acts described herein are replaceable by equivalents which perform the same function, even if the structure or acts are different, as known in the art. Therefore, the scope of the invention is limited only by the elements and limitations as used in the claims. When used in the following claims, the terms "comprise", "include", "have" and their conjugates mean "including but not limited to".

**Claims**

1. An ultrasonic generator, comprising:

    a source (508, 510) of pulsed electro-magnetic radiation that generates radiation having a plurality of different wavelengths;
    an electromagnetic waveguide (100, 200, 302, 304, 400, 514, 802) coupled to the source; and
    at least one absorbing region (106, 206, 108, 404, 604) in said waveguide (100, 200, 302, 304, 400, 514, 802) that converts incident electromagnetic radiation of fewer than all the plurality of generated wavelengths from the source into ultrasonic waves.

2. A generator according to claim 1, wherein at least one of the wavelengths not converted by the absorbing region (106, 206, 108, 404, 604) into electromagnetic radiation is used for light illumination.

3. A generator according to claim 1 or claim 2, wherein said waveguide (100, 200, 302, 304, 400, 514, 802) is formed into a guidewire (600).

4. A generator according to any of claims 1-3, wherein said generator is adapted to be inserted into a body.

5. A generator according to any of claims 1-4, wherein said waveguide (100, 200, 302, 304,400,514, 802) comprises an optical fiber.

6. A generator according to claim 5, wherein said fiber includes a non-acoustic optical fiber sensor.

7. A generator according to any of claims 1-6, wherein said absorbing region (106, 206, 108, 404, 604) is optically controllable to change its absorption characteristics.

8. A generator according to any of claims 1-7, wherein said at least one absorbing region (106, 206, 108, 404, 604) comprises at least two absorbing regions.

9. A generator according to claim 8, wherein said at least two regions have same absorbing characteristics.

10. A generator according to claim 8, wherein said at least two regions have different absorbing characteristics.

11. A generator according to any of claims 8-10, wherein said at least two regions have same selectivity.

12. A generator according to any of claims 1-11, wherein said absorbing region (106, 206, 108, 404. 604) comprises a volume absorber (206) that absorbs said radiation along its length in a direction of propagation of said radiation.

13. A generator according to claim 12, wherein said volume absorber has axially uniform absorption characteristics, along the axis of said waveguide(100, 200, 302, 304, 400, 514; 802).

14. A generator according to claim 12, wherein said volume absorber has axially non-uniform absorption characteristics, along the axis of said waveguide(100, 200, 302, 304, 400, 514, 802).

15. A generator according to claim 14, wherein said volume absorber has stepped absorption characteristics, along the axis of said waveguide(100, 200, 302, 304, 400, 514, 802).

16. A generator according to any of claims 1-15, wherein said absorbing region (106, 206, 206, 108, 404, 604) comprises a solid absorber.

17. A generator according to any of claims 1-16, wherein said absorbing region comprises a fluid absorber.

18. A generator according to any of claims 1-17, wherein said waveguide (100, 200, 302, 304, 400, 514, 802) comprises an acousto-optical modulator portion that modulates light waves responsive to an acoustic field.

19. A generator according to claim 18, comprising an optical detector coupled to said waveguide (100, 200, 302, 304, 400, 514, 802) which generates a signal responsive to said acoustic field.

20. A generator according to claim 19, comprising a signal processor that reconstructs an image from said signal.

21. A generator according to claim 20, comprising a signal processor operative to reconstruct a tissue characterization from said signal.

22. A generator according to claim 21 comprising a signal processor operative to reconstruct a distance from said signal.

23. A generator according to any of claims 1-22, wherein said source provides at least one wavelength having a high power level that passes through said absorbing region substantially unabsorbed.

24. A generator according to any of claims 1-23, comprising a plurality of waveguides (100, 200, 302, 304, 400, 514, 802) arranged in a phased-array and a controller that controls said source to activate said array as a pnased-array.

25. A generator according to any of claims 1-24, wherein said ultrasonic wave is operative to be steered in space by said generator without moving the absorbirig region.

26. A generator according to any of claims 1-25, comprising a controller (506) operative to control said source.

27. A generator according to claim 26, wherein said controller synchronizes an operation of said generator with a separate treatment and/or imaging device.

28. A generator according to claim 12 comprising a reflector (110, 210) for reflecting at least a portion of the light that passes once through said absorber, to pass at least a second time through said absorber.

29. A generator according to claim 28, comprising a second reflector (212) for reflecting at least a portion of the light that passes twice through said absorber, to pass at least a third time through said absorber.

30. A generator according to any of the preceding claims, wherein a second one of said wavelengths interacts with said waveguide (100, 200, 302, 304, 400, 514, 802) other than at said at least one absorbing region, to generate ultrasound.

31. A generator according to claim 30, wherein said second generated ultrasound has an intensity high enough to attack adjacent plaque in a blood vessel.

**Patentansprüche**

1. Ultraschall-Erzeugungsvorrichtung, die umfasst:

eine Quelle (508, 510) gepulster elektromagnetischer Strahlung, die Strahlung erzeugt, die eine Vielzahl verschiedener Wellenlängen hat;

einen elektromagnetischen Wellenleiter (100, 200, 302, 304, 400, 514, 802), der mit der Quelle gekoppelt ist; und wenigstens einen absorbierenden Bereich (106, 206, 108, 404, 604) in dem Wellenleiter (100, 200, 302, 304, 400, 514, 802), der auftreffende elektromagnetische Strahlung von weniger als allen der Vielzahl erzeugten Wellenlängen der Quelle in Ultraschallwellen umwandelt.

2. Erzeugungsvorrichtung nach Anspruch 1, wobei wenigstens eine der Wellenlängen, die durch den absorbierenden Bereich (106, 206, 108, 404, 604) nicht in elektromagnetische Strahlung umgewandelt wird, zur Beleuchtung mit Licht verwendet wird.

3. Erzeugungsvorrichtung nach Anspruch 1 oder Anspruch 2, wobei der Wellenleiter (100, 200, 302, 304, 400, 514, 802) als ein Führungsdraht (600) ausgebildet ist.

4. Erzeugungsvorrichtung nach einem der Ansprüche 1-3, wobei die Erzeugungsvorrichtung zum Einführen in einen Körper eingerichtet ist.

5. Erzeugungsvorrichtung nach einem der Ansprüche 1-4, wobei der Wellenleiter (1000, 200, 302, 304, 400, 514, 802) eine Lichtleitfaser umfasst.

6. Erzeugungsvorrichtung nach Anspruch 5, wobei die Faser einen nicht akustischen Lichtleitfasersensor enthält.

7. Erzeugungsvorrichtung nach einem der Ansprüche 1-6, wobei der absorbierende Bereich (106, 206, 108, 404, 604) optisch gesteuert werden kann, um seine Absorptionseigenschaften zu ändern.

8. Generator nach einem der Ansprüche 1-7, wobei der wenigstens eine absorbierende Bereich wenigstens zwei absorbierende Bereiche umfasst.

9. Erzeugungsvorrichtung nach Anspruch 8, wobei die wenigstens zwei Bereiche gleiche Absorptionseigenschaften haben.

10. Erzeugungsvorrichtung nach Anspruch 8, wobei die wenigstens zwei Bereiche verschiedene Absorptionseigenschaften haben.

11. Erzeugungsvorrichtung nach einem der Ansprüche 8-10, wobei die wenigstens zwei Bereiche gleiche Selektivität haben.

12. Erzeugungsvorrichtung nach einem der Ansprüche 1-11, wobei der absorbierende Bereich (106, 206, 108, 404, 604) einen Volumenabsorber (206) umfasst, der die Strahlung über seine Länge in einer Ausbreitungsrichtung der Strahlung absorbiert.

13. Erzeugungsvorrichtung nach Anspruch 12, wobei der Volumenabsorber axial einheitliche Absorptionseigenschaften entlang der Achse des Wellenleiters (100, 200, 302, 304, 400, 514, 802) hat.

14. Erzeugungsvorrichtung nach Anspruch 12, wobei der Volumenabsorber axial uneinheitliche Absorptionseigenschaften entlang der Achse des Wellenleiters (100, 200, 302, 304, 400, 514, 802) hat.

15. Erzeugungsvorrichtung nach Anspruch 14, wobei der Volumenabsorber abgestufte Absorptionseigenschaften entlang der Achse des Wellenleiters (100, 200, 302, 304, 400, 514, 802) hat.

16. Erzeugungsvorrichtung nach einem der Ansprüche 1-15, wobei der absorbierende Bereich (106, 206, 206, 108, 404, 604) einen Festabsorber umfasst.

17. Erzeugungsvorrichtung nach einem der Ansprüche 1-16, wobei der absorbierende Bereich einen Fluidabsorber umfasst.

18. Erzeugungsvorrichtung nach einem der Ansprüche 1-17, wobei der Wellenleiter (100, 200, 302, 304, 400, 514, 802) einen akustisch-optischen Modulatorabschnitt umfasst, der Lichtwellen in Reaktion auf ein akustisches Feld moduliert.

19. Erzeugungsvorrichtung nach Anspruch 18, die einen optischen Detektor umfasst, der mit dem Wellenleiter (100, 200, 302, 304, 400, 514, 802) gekoppelt ist und in Reaktion auf das akustische Feld ein Signal erzeugt.

20. Erzeugungsvorrichtung nach Anspruch 19, die einen Signalprozessor umfasst, der aus dem Signal ein Bild rekonstruiert.

21. Erzeugungsvorrichtung nach Anspruch 20, die einen Signalprozessor umfasst, der in Funktion aus dem Signal eine Gewebecharakterisierung rekonstruiert.

22. Erzeugungsvorrichtung nach Anspruch 21, die einen Signalprozessor umfasst, der in Funktion aus dem Signal einen Abstand rekonstruiert.

23. Erzeugungsvorrichtung nach einem der Ansprüche 1-22, wobei die Quelle wenigstens eine Wellenlänge mit einem hohen Energiepegel bereitstellt, die im Wesentlichen unabsorbiert durch den absorbierenden Bereich hindurch tritt.

24. Erzeugungsvorrichtung nach einem der Ansprüche 1-23, die eine Vielzahl von Wellenleitern (100, 200, 302, 304, 400, 514, 802), die in einer phasengesteuerten Anordnung angeordnet sind, sowie eine Steuereinrichtung umfasst, die die Quelle so steuert, dass sie die Anordnung als eine phasengesteuerte Anordnung aktiviert.

25. Erzeugungsvorrichtung nach einem der Ansprüche 1-24, wobei die Ultraschallwelle in Funktion räumlich durch die Erzeugungsvorrichtung gesteuert wird, ohne den absorbierenden Bereich zu bewegen.

26. Erzeugungsvorrichtung nach einem der Ansprüche 1-25, die eine Steuereinrichtung (506) umfasst, die in Funktion die Quelle steuert.

27. Erzeugungsvorrichtung nach Anspruch 26, wobei die Steuereinrichtung eine Funktion der Erzeugungsvorrichtung mit einer separaten Behandlungs- und/oder Bilderzeugungseinrichtung synchronisiert.

28. Erzeugungsvorrichtung nach Anspruch 12, die einen Reflektor (110, 210) umfasst, der wenigstens einen Teil des Lichtes reflektiert, das einmal durch den Absorber hindurch tritt, so dass es wenigstens ein zweites Mal durch den Absorber hindurch tritt.

29. Erzeugungsvorrichtung nach Anspruch 28, die einen zweiten Reflektor (212) umfasst, der wenigstens einen Teil des Lichtes reflektiert, das zweimal durch den Absorber hindurch tritt, so dass es wenigstens ein drittes Mal durch den Absorber hindurch tritt.

30. Erzeugungsvorrichtung nach einem der vorangehenden Ansprüche, wobei eine zweite der Wellenlängen mit dem Wellenleiter (100, 200, 302, 304, 400, 514, 802) außer in dem wenigstens einen absorbierenden Bereich in Wechselwirkung tritt, um Ultraschall zu erzeugen.

31. Erzeugungsvorrichtung nach Anspruch 30, wobei der zweite erzeugte Ultraschall eine Intensität hat, die hoch genug ist, um angrenzenden Plaque in einem Blutgefäß anzugreifen.

**Revendications**

1. Générateur d'ultrasons, comportant :

   une source (508, 510) de rayonnement électromagnétique pulsé qui génère un rayonnement ayant une pluralité de longueurs d'onde différentes,
   un guide d'ondes électromagnétiques (100, 200, 302, 304, 400, 514, 802) couplé à la source, et
   au moins une région absorbante (106, 206, 108, 404, 604) dans ledit guide d'ondes (100, 200, 302, 304, 400, 514, 802) qui convertit un rayonnement électromagnétique incident de moins de la totalité de la pluralité de longueurs d'onde générées par la source en ondes ultrasonores.

2. Générateur selon la revendication 1, dans lequel au moins l'une des longueurs d'onde non-converties par la région absorbante (106, 206, 108, 404, 604) en rayonnement électromagnétique est utilisée pour un éclairement lumineux.

3. Générateur selon la revendication 1 ou la revendication 2, dans lequel ledit guide d'ondes (100, 200, 302, 304, 400, 514, 802) est formé en fil guide (600).

4. Générateur selon l'une quelconque des revendications 1 à 3, dans lequel ledit générateur est adapté pour être inséré dans un corps.

5. Générateur selon l'une quelconque des revendications 1 à 4, dans lequel ledit guide d'ondes (100, 200, 302, 304, 400, 514, 802) comporte une fibre optique.

6. Générateur selon la revendication 5, dans lequel ladite fibre inclut un capteur de fibre optique non-acoustique.

7. Générateur selon l'une quelconque des revendications 1 à 6, dans lequel ladite région absorbante (106, 206, 108, 404, 604) est optiquement contrôlable pour changer ses caractéristiques d'absorption.

8. Générateur selon l'une quelconque des revendications 1 à 7, dans lequel ladite au moins une région absorbante (106, 206, 108, 404, 604) comporte au moins deux régions absorbantes.

9. Générateur selon la revendication 8, dans lequel lesdites au moins deux régions ont les mêmes caractéristiques d'absorption.

10. Générateur selon la revendication 8, dans lequel lesdites au moins deux régions ont des caractéristiques d'absorption différentes.

11. Générateur selon l'une quelconque des revendications 8 à 10, dans lequel lesdites au moins deux régions ont la même sélectivité.

12. Générateur selon l'une quelconque des revendications 1 à 11, dans lequel ladite région absorbante (106, 206, 108, 404, 604) comporte un absorbeur volumique (206) qui absorbe ledit rayonnement sur sa longueur dans une direction de propagation dudit rayonnement.

13. Générateur selon la revendication 12, dans lequel ledit absorbeur volumique a une caractéristique d'absorption axialement uniforme, le long de l'axe dudit guide d'ondes (100, 200, 302, 304, 400, 514, 802).

14. Générateur selon la revendication 12, dans lequel ledit absorbeur volumique a une caractéristique d'absorption axialement non-uniforme, le long de l'axe dudit guide d'ondes (100, 200, 302, 304, 400, 514, 802).

15. Générateur selon la revendication 14, dans lequel ledit absorbeur volumique a une caractéristique d'absorption étagée, le long de l'axe dudit guide d'ondes (100, 200, 302, 304, 400, 514, 802).

16. Générateur selon l'une quelconque des revendications 1 à 15, dans lequel ladite région absorbante (106, 206, 108, 404, 604) comporte un absorbeur solide.

17. Générateur selon l'une quelconque des revendications 1 à 16, dans lequel ladite région absorbante comporte un absorbeur fluide.

18. Générateur selon l'une quelconque des revendications 1 à 17, dans lequel ledit guide d'ondes (100, 200, 302, 304, 400, 514, 802) comporte une partie de modulation acousto-optique qui module des ondes lumineuses en réponse à un champ acoustique.

19. Générateur selon la revendication 18, comportant un détecteur optique couplé audit guide d'ondes (100, 200, 302, 304, 400, 514, 802) qui génère un signal en réponse audit champ acoustique.

20. Générateur selon la revendication 19, comportant un processeur de signal qui reconstitue une image d'après ledit signal.

21. Générateur selon la revendication 20, comportant un processeur de signal opérationnel pour reconstituer une caractérisation de tissu d'après ledit signal.

22. Générateur selon la revendication 21, comportant un processeur de signal opérationnel pour reconstituer une distance d'après ledit signal.

23. Générateur selon l'une quelconque des revendications 1 à 22, dans lequel ladite source fournit au moins une longueur d'onde ayant un niveau de puissance élevé qui passe à travers ladite région absorbante sensiblement non-absorbée.

24. Générateur selon l'une quelconque des revendications 1 à 23, comportant une pluralité de guides d'ondes (100, 200, 302, 304, 400, 514, 802) disposés en un réseau phasé et un contrôleur qui commande ladite source pour activer ledit réseau en tant que réseau phasé.

25. Générateur selon l'une quelconque des revendications 1 à 24, dans lequel ladite onde ultrasonore est opérationnelle pour être dirigée dans l'espace par ledit générateur sans déplacer la région absorbante.

26. Générateur selon l'une quelconque des revendications 1 à 25, comportant un contrôleur (506) opérationnel pour commander ladite source.

27. Générateur selon la revendication 26, dans lequel ledit contrôleur synchronise une opération dudit générateur à l'aide d'un dispositif de traitement et/ou d'imagerie séparé.

28. Générateur selon la revendication 12, comportant un réflecteur (110, 210) pour refléchir au moins une partie de la lumière qui passe une fois à travers ledit absorbeur, pour passer au moins une deuxième fois à travers ledit absorbeur.

29. Générateur selon la revendication 28, comportant un second réflecteur (212) pour refléchir au moins une partie de la lumière qui passe deux fois à travers ledit absorbeur, pour passer au moins une troisième fois à travers ledit absorbeur.

30. Générateur selon l'une quelconque des revendications précédentes, dans lequel une deuxième desdites longueurs d'onde interagit avec ledit guide d'ondes (100, 200, 302, 304, 400, 514, 802) autre qu'à ladite au moins une région absorbante, pour générer un ultrason.

31. Générateur selon la revendication 30, dans lequel ledit second ultrason généré a une intensité suffisamment élevée pour attaquer une plaque adjacente dans un vaisseau sanguin.

FIG.1

FIG.2A

HEAT DISTRIBUTION IN 2-PASS UNIFORM ABSORBER

FIG.2B

HEAT DISTRIBUTION IN 1-PASS EXP. ABSORBER

FIG.2C

HEAT DISTRIBUTION IN 1-PASS STEPPED ABSORBER

FIG.2D

FIG.3A

FIG.3B

FIG.4

EP 1 471 848 B1

FIG.5

FIG.6

EP 1 471 848 B1

FIG.7

FIG.8

FIG.9